**Europäisches Patentamt**

**European Patent Office**

**Office européen des brevets**

(19)

(11) Veröffentlichungsnummer: **0 108 383 B1**

(12) **EUROPÄISCHE PATENTSCHRIFT**

(45) Veröffentlichungstag der Patentschrift:
31.01.90

(51) Int. Cl.⁴: **A 61 N 1/36,** A 61 B 17/56

(21) Anmeldenummer: 83110916.0

(22) Anmeldetag: 02.11.83

(54) Implantierbare Vorrichtung zur Stimulation des Knochenwachstums.

(30) Priorität: 03.11.82 DE 3240592

(43) Veröffentlichungstag der Anmeldung:
16.05.84 Patentblatt 84/20

(45) Bekanntmachung des Hinweises auf die Patenterteilung:
31.01.90 Patentblatt 90/5

(84) Benannte Vertragsstaaten:
AT BE CH DE FR GB IT LI LU NL SE

(56) Entgegenhaltungen:
EP-A- 0 001 289
DE-A- 2 636 818
DE-A- 3 132 488

(73) Patentinhaber: Kraus, Werner, Augustenstrasse 41,
D-8000 München 2 (DE)

(72) Erfinder: Kraus, Werner, Augustenstrasse 41,
D-8000 München 2 (DE)

(74) Vertreter: von Bezold, Dieter, Dr. et al, Dr. Dieter von
Bezold Dipl.-Ing. Peter Schütz Dipl.-Ing. Wolfgang
Heusler Brienner Strasse 52, D-8000 München 2 (DE)

ACTORUM AG

## Beschreibung

Die vorliegende Erfindung betrifft eine Vorrichtung, wie sie im Oberbegriff des Patentanspruchs 1 im Hinblick auf DE-A-2 311 817 als bekannt vorausgesetzt wird. Insbesondere betrifft die Erfindung ein Implantat zur Heilung von Knochenschäden durch niederfrequente elektrische Ströme, die durch Induktion erzeugt werden und eine Frequenz haben, die vorzugsweise unter 30 Hz liegt.

Der vorliegenden Erfindung liegt die Aufgabe zugrunde, die oben genannte bekannte Vorrichtung dahingehend weiterzubilden, daß sie vielseitiger und bequemer anwendbar ist und sich insbesondere für die Heilung von größeren Knochendefekten (z.B. Diastasen bis 10 cm und mehr) eignet.

Diese Aufgabe wird erfindungsgemäß durch eine Vorrichtung mit einer Aufnehmerspule, die mindestens einen ersten und einen zweiten Anschluß aufweist; einer die Aufnehmerspule umgebenden länglichen Umhüllung aus gewebeverträglichem Material; mindestens einer zum Anschließen einer Gewebeelektrode dienenden Anschlußvorrichtung, welche über einen isolierten Draht elektrisch mit dem ersten Anschluß der Aufnehmerspule verbunden ist, gelöst, die dadurch gekennzeichnet ist, daß der zweite Anschluß der Aufnehmerspule mit einer langgestreckten, streifenförmigen Gewebeelektrode gekoppelt ist, die in Längsrichtung der Umhüllung verläuft und im wesentlichen senkrecht von dieser vorspringt.

Weiterbildungen und vorteilhafte Ausgestaltungen der erfindungsgemäßen Vorrichtung sind Gegenstand von Unteransprüchen.

Bei der vorliegenden Vorrichtung können als Elektroden nach Wahl Knochenschrauben und/ oder Kirschnerdrähte und/oder leicht anbringbare, im wesentlichen axial zur Aufnehmerspulenanordnung verlaufende streifenförmige Elektroden («Kielelektroden») verwendet werden. Die Elektrodenstruktur zur Applikation des durch das niederfrequente elektromagnetische Feld induzierten niederfrequenten Wechselstromes läßt sich also an die jeweilige Situation sehr gut anpassen.

Im folgenden wird ein bevorzugtes Ausführungsbeispiel der Erfindung unter Bezugnahme auf die Zeichnung näher erläutert.

Es zeigen:

Fig. 1 eine vereinfachte, etwas schematisierte Draufsicht auf eine Vorrichtung gemäß einer Ausführungsform der Erfindung;

Fig. 2 einen Querschnitt in einer Ebene II–II der Fig. 1;

Fig. 3 eine Draufsicht auf eine aufschiebbare streifenförmige Elektrode («Kielelektrode»);

Fig. 4 einen Querschnitt in einer Ebene IV–IV der Fig. 3;

Fig. 5 eine perspektivische Ansicht einer Vorrichtung gemäß Fig. 1 mit aufgesetzten Kielelektroden gemäß Fig. 3 und 4 sowie zusätzlichen Kirschnerdraht-Elektroden;

Fig. 6 eine perspektivische Ansicht einer bevorzugten Ausführungsform der Erfindung und

Fig. 7 eine Draufsicht auf die Unterseite der Vorrichtung gemäß Fig. 6.

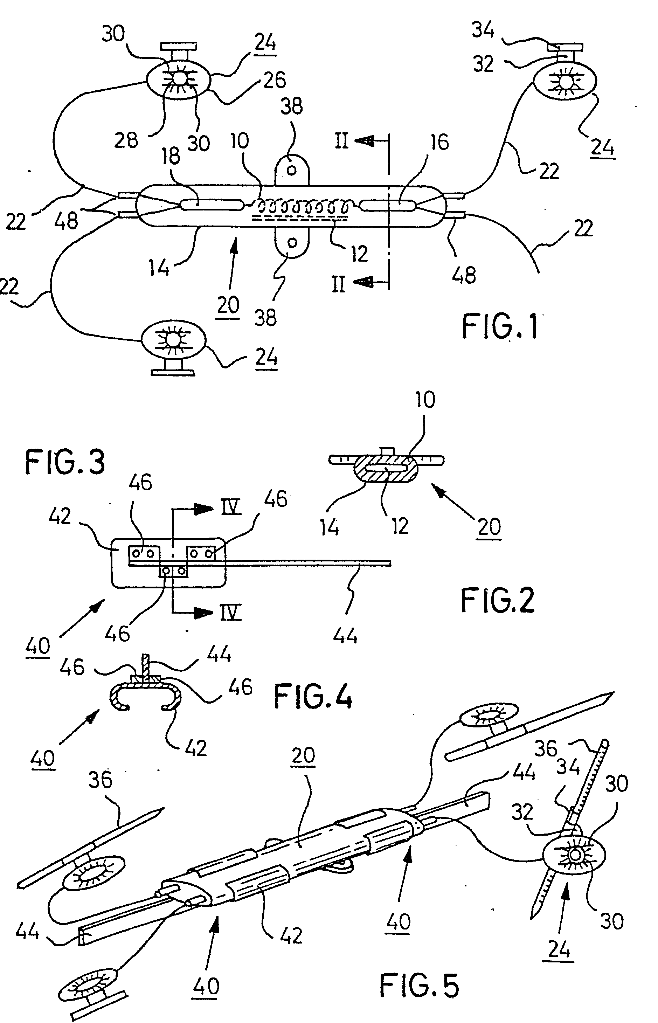

Die in den Figuren 1 bis 5 dargestellte implantierbare Vorrichtung enthält eine implantierbare solenoidförmige Aufnehmerspule 10 mit einem Magnetkern 12, die in Fig. 1 nur als Schaltbild dargestellt sind. Die Aufnehmerspule 10 und der Magnetkern 12 sind mit einer Hülle 14 aus einem gewebeverträglichen Isoliermaterial, z.B. PTFE, umgeben.

Die Enden der Aufnehmerspule 10 sind elektrisch jeweils mit einem länglichen Kontakt 16 bzw. 18 verbunden, die mit frei liegender Oberfläche auf der einen Seite der aus der Aufnehmerspule 10, dem Magnetkern 12 und der Umhüllung 14 bestehenden Aufnehmerspulenanordnung 20 angeordnet sind und in Längsrichtung von dieser Anordnung verlaufen, wie aus Fig. 1 ersichtlich ist. Die Enden der Spule sind außerdem jeweils mit zwei isolierten Anschlußdrähten 22 verbunden, die jeweils zu einer Anschlußvorrichtung 24 führen. Die Anschlußvorrichtungen 24 bestehen aus einem gewebeverträglichen elektrisch leitenden Material, wie Osteosynthesemetall, und enthalten einen länglichen, tellerförmigen Teil 26 mit einem Loch 28 für eine Knochenschraube. Der das Loch umgebende Bereich ist muldenförmig vertieft, um den Schraubenkopf aufnehmen zu können, außerdem sind auf diametral entgegengesetzten Seiten des Loches 28 zwei tangentiale parallele Schlitze 30 vorgesehen, so daß Knochenschrauben unterschiedlicher Dicken eingesetzt werden können. Die Anschlußvorrichtungen 24 weisen ferner jeweils eine seitliche Lasche 32 auf, an die sich ein hülsenförmig gerollter Teil anschließt, in dem ein Kirschnerdraht 36 (Fig. 5) eingesetzt werden kann.

Die Umhüllung 14 ist mit seitlichen, durchbrochenen, laschenförmigen Ansätzen 38 versehen, mit der die Aufnehmerspulenanordnung 20 im Implantationsbereich durch einen Draht oder dergleichen fixiert werden kann.

Die Kontakte 16, 18 dienen jeweils zum elektrischen Anschluß einer Elektrodenanordnung 40, die in Fig. 3 und 4 dargestellt ist und einen der Querschnittsform der Aufnehmerspulenanordnung 20 angepaßten klammerartigen Teil 42 sowie einen streifenförmigen Elektrodenteil 44 enthält. Der Elektrodenteil 44 ist z.B. über laschenartige Ansätze 46 durch Punktschweißen mit dem klammerartigen Teil 42 verbunden. Die Elektrodenanordnung 40 kann in Längsrichtung derart auf die Aufnehmerspulenanordnung 20 aufgeschoben werden, daß der Kontakt 16 bzw. 18 eine elektrische Verbindung zwischen der Aufnehmerspule einerseits und dem klammerartigen Teil 42 und damit mit der streifenförmigen Elektrode 44 («Kielelektrode») herstellt, die bei montierter Elektrodenanordnung 40 auf der einen Seite der Aufnehmerspulenanordnung 20 axial zu dieser verläuft und über ihre Enden vorspringt, wie aus Fig. 5 ersichtlich ist. Der streifenförmige Elektrodenteil 44 bildet eine Gewebeelektrode, die im montierten Zustand von der Umhüllung 14 im wesentlichen senkrecht vorspringt, also «hochkant» auf dem betreffenden Teil der Außenseite der Umhüllung steht.

Die Umhüllung 14 kann dort, wo die isolierten Anschlußdrähte 22 austreten, mit zapfenförmigen Verlängerungen 48 versehen sein, die als Knickschutz dienen.

Bei der Verwendung der Vorrichtung wird diese im Bereich des zu heilenden Knochenschadens implantiert. Auch größere Diastasen können mittels der streifenförmigen Elektroden 44 überbrückt werden. Je nach Bedarf kann eine beliebige Anzahl der Anschlußvorrichtungen 24 mit Knochenschrauben-Elektroden und/oder Kirschnerdraht-Elektroden versehen werden. Die aus einem relativ dünnen Blech bestehenden Laschen 32 lassen sich leicht verbiegen und verdrehen, so daß der eingesetzte Kirschnerdraht 36 mit der gewünschten Orientierung implantiert werden kann, wie beispielsweise in Fig. 5 rechts unten dargestellt ist.

Die streifenförmigen Elektroden 44 können durch Zuschneiden auf die gewünschte Länge gebracht werden. Sie lassen sich außerdem biegen, falls dies erwünscht ist. Soweit die Anschlußvorrichtungen 24 nicht benötigt werden, können sie zusammen mit dem zugehörigen Anschlußdraht an der Verlängerung 48 einfach abgeschnitten werden.

Durch die Schlitze 30 können die Anschlußvorrichtungen für Schrauben verschiedener Durchmesser, z.B. Schrauben mit Durchmessern zwischen 3,0 und 4,5 mm verwendet werden. Die Hülse 34 hat normalerweise einen Innendurchmesser von etwa 2 mm, da sie jedoch nicht geschlossen ist, kann sie auch dickere Kirschnerdrähte aufnehmen.

Die Aufnehmerspule 10 und der Magnetkern 12 können in bekannter Weise bemessen sein. Nach der Implantation wird wie bei der oben erwähnten bekannten Vorrichtung in der Aufnehmerspule 10 ein niederfrequenter Wechselstrom induziert.

Die Aufnehmerspulenanordnung 20 kann in der Praxis einen Querschnitt in Form eines flachen Rechtecks mit abgerundeten Ecken haben, dessen lange Seite ca. 12 mm und dessen kurze Seite ca. 6 mm groß sind. Die Länge der Aufnehmerspulenanordnung 20 ohne die Anschlußdrähte kann etwa 75 mm betragen. Die streifenförmigen Elektroden 44, die, wie dargestellt, vorzugsweise senkrecht auf den flachen Seiten des durch die Aufnehmerspulenanordnung gebildeten, näherungsweise streifenförmigen Körpers stehen, können etwa 60 bis 80 mm lang, etwa 4 bis 7 mm breit und etwa 0,5 mm dick sein. Die Elektrodenanordnung 40 besteht vorzugsweise aus gewebeverträglichem Osteosynthesemetall, insbesondere einer üblichen Kobalt-Chromlegierung.

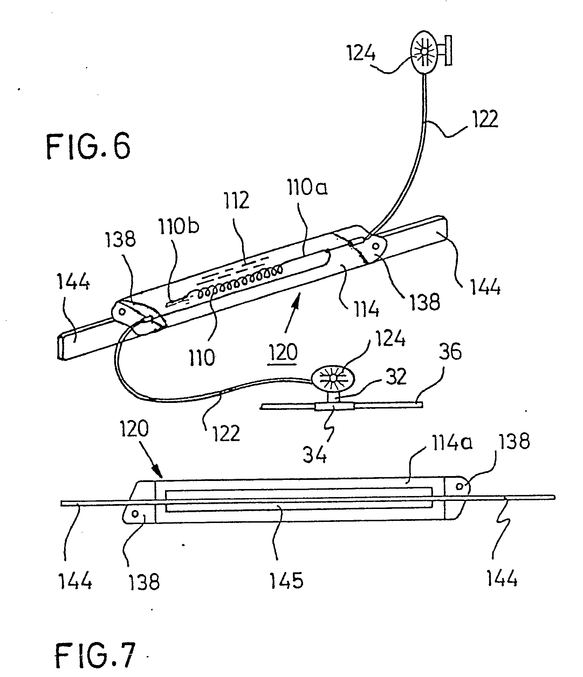

Die in den Figuren 6 und 7 dargestellte bevorzugte Ausführungsform der implantierbaren Vorrichtung gemäß der Erfindung enthält eine Aufnehmerspulenanordnung 120, die im wesentlichen wie die Aufnehmerspulenanordnung 20 gemäß Fig. 1 ausgebildet ist und eine nur als Schaltbild dargestellte Aufnehmerspule 110 mit Magnetkern 112 sowie eine Umhüllung 114 für diese nur schematisch angedeuteten Bauteile enthält. Die Umhüllung ist an ihren Längsenden mit Befestigungslaschen 138 versehen. Die isolierten Drähte 122 mit den Anschlußvorrichtungen 24 sind bei dieser Ausführungsform mit dem gleichen Anschluß 110a der Aufnehmerspule 110 elektrisch verbunden. Der andere Anschluß 110b ist permanent mit einer durchgehenden, streifenförmigen Gewebeelektrode 144 verbunden, die sich in Längsrichtung der Aufnehmerspulenanordnung 120 erstreckt, mit dieser fest verbunden ist, hochkant auf ihr steht und in Axialrichtung über die Enden der Aufnehmerspulenanordnung hinausreicht, wie insbesondere aus Fig. 7 ersichtlich ist. Die Aufnehmerspulenanordnung 120 hat eine abgeflachte Unterseite 114a, auf der sich eine zusätzliche plattenförmige Elektrode 145 befindet, die elektrisch und mechanisch mit der kielartigen Gewebeelektrode 144 verbunden ist, und eine zusätzliche Kontaktfläche am Außenmantel der Anordnung 120 bildet.

Die mechanisch fest mit der Anordnung 110 verbundene kielartige Gewebeelektrode 144 und die zusätzliche Kontaktfläche 145 bilden die eine Elektrode, während die Anschlußvorrichtungen 124 mit den an ihnen angebrachten Elektroden, wie einem Kirschnerdraht 36 oder einer Knochenschraube (nicht dargestellt) die Gegenpole bilden. Die streifenförmige Elektrode 144 kann in der Praxis z.B. 120 mm lang sein und bei Benutzung auf die zur Überbrückung eines Knochendefektes benötigte Länge zugeschnitten werden.

Die Ausführungsform gemäß Fig. 6 und 7 kann dadurch abgewandelt werden, daß die Elektrodenanordnung 144–145 quer zur Längsrichtung der Anordnung 120 in zwei elektrisch getrennte Teile geteilt wird, die ähnlich, wie bei Fig. 5 jeweils eine hochkant stehende, streifenförmige Gewebeelektrode enthalten, die über ein Ende der Anordnung 120 hinaus vorspringt. Die beiden Teile der Elektrodenanordnung sind dann mit verschiedenen Anschlüssen der Aufnehmerspule 110 verbunden. Der Teil der streifenförmigen Elektrode und der Anschlußdraht 122 für die Anschlußvorrichtung 24, die sich am gleichen axialen Ende der Vorrichtung 120 befinden, sind dann vorzugsweise mit verschiedenen Anschlüssen der Aufnehmerspule verbunden.

Die die zusätzliche Kontaktfläche bildende Platte 145 ist vorzugsweise mechanisch fest mit der streifenförmigen Gewebeelektrode 144 verbunden und unterstützt die Verankerung dieser Elektrode in der Anordnung 120.

## Patentansprüche

1. Implantierbare Vorrichtung zur Stimulation des Knochenwachstums, mit einer Aufnehmerspule (10), die mindestens einen ersten und einen zweiten Anschluß aufweist; einer die Aufnehmerspule (10) umgebenden länglichen Umhüllung (14) aus gewebeverträglichem Material; mindestens einer zum Anschließen einer Gewebeelektrode (36) dienenden Anschlußvorrichtung (24), welche über einen isolierten Draht (22) elektrisch mit dem ersten Anschluß der Aufnehmerspule (10) verbunden ist, dadurch gekennzeichnet, daß der zweite Anschluß der Aufnehmerspule (10) mit einer langgestreckten, streifenförmigen Gewebeelektrode (44) gekoppelt ist, die in Längsrichtung der Umhüllung (14) verläuft und im wesentlichen senkrecht von dieser vorspringt.

2. Vorrichtung nach Anspruch 1, dadurch gekennzeichnet, daß die Gewebeelektrode (44) über die Längsenden der Umhüllung hinausreicht.

3. Vorrichtung nach Anspruch 1 oder 2, dadurch gekennzeichnet, daß die Gewebeelektrode (44) mechanisch fest mit der aus Aufnehmerspule (10) und deren Umhüllung (14) gebildeten Anordnung verbunden ist.

4. Vorrichtung nach einem der Ansprüche 1 bis 3, dadurch gekennzeichnet, daß die Umhüllung näherungsweise die Form eines flachen Rechteckes hat und daß die Ebene der streifenförmigen Gewebeelektrode (44) senkrecht zu den großen Seiten des Rechteckes verläuft.

5. Vorrichtung nach einem der vorhergehenden Ansprüche, dadurch gekennzeichnet, daß an der Außenseite des Teiles der Umhüllung (14) von dem aus die streifenförmige Gewebeelektrode vorspringt, eine mit dieser elektrisch verbundene plattenförmige Elektrode angeordnet ist.

6. Vorrichtung nach Anspruch 1 oder 2, dadurch gekennzeichnet, daß der zweite Anschluß der Aufnehmerspule (10) mit einem freiliegenden, auf einer Seite der Umhüllung (14) angeordneten Kontakt (18) verbunden ist und daß eine auf die Umhüllung (14) aufschiebbare Elektrodenanordnung (40) vorgesehen ist, die einen ersten elektrisch leitenden Teil (42), der im aufgeschobenen Zustand der Elektronenanordnung (40) den Kontakt (18) berührt, und einen mit dem ersten Teil elektrisch und mechanisch verbundenen zweiten Teil (44) enthält, der die streifenförmige Gewebeelektrode bildet.

7. Vorrichtung nach Anspruch 6, dadurch gekennzeichnet, daß die Anschlußvorrichtung (24) einen hülsenförmigen Teil (34) zur Aufnahme einer stabförmigen Elektrode (36) enthält, der über einen laschenförmigen, leicht biegsamen Teil (32) mit einem das Schraubenloch (28) enthaltenden Hauptteil der Anschlußvorrichtung verbunden ist.

8. Vorrichtung gemäß dem Oberbegriff des Anspruchs 1 oder nach einem der Ansprüche 1 bis 7, dadurch gekennzeichnet, daß die Anschlußvorrichtung (24) einen hülsenförmigen Teil (34) zur Aufnahme einer stabförmigen Elektrode (36) enthält, der über einen laschenförmigen, leicht biegsamen Teil (32) mit einem ein Schraubenloch (28) enthaltenden Hauptteil der Anschlußvorrichtung verbunden ist.

## Claims

1. Implantable device for the stimulation of bone growth, with a pick-up coil (10) having at least a first and a second terminal; an elongated envelope (14) of tissue-compatible material surrounding the pick-up coil (10); at least one connection device (24), for making the connection to a tissue electrode (36), which through an insulated wire (22) is electrically connected to the first terminal of the pick-up coil (10), characterised in that the second terminal of the pick-up coil (10) is coupled to a longitudinally extended, strip-like tissue electrode (44) which runs in the longitudinal direction of the envelope (14) and protrudes from this in an essentially perpendicular direction.

2. Device according to claim 1, characterised in that the tissue electrode (44) extends beyond the longitudinal ends of the envelope.

3. Device according to claim 1 or 2, characterised in that the tissue electrode (44) is firmly connected mechanically with the pick-up coil (10) and the arrangement forming its envelope (14).

4. Device according to one of the claims 1 to 3, characterised in that the envelope approximately has the shape of a flat rectangle and that the plane of the strip-like tissue electrode (44) extends perpendicularly to the large sides of the rectangle.

5. Device according to one of the aforementioned claims, characterised in that on the outside of the part of the envelope (14) from which the strip-like tissue electrode protrudes, there is arranged a plate-shaped electrode which is electrically connected with the tissue electrode.

6. Device according to claim 1 or 2, characterised in that the second connection of the pick-up coil (10) is connected to an exposed contact (18) arranged on one side of the envelope (14), and for sliding onto the casing (14) is provided an electrode arrangement (40) which comprises a first electrically conducting part (42), which touches the contact (18) when in the slid-on position, and a second part (44), forming the strip-like tissue electrode, which is mechanically and electrically connected with the first part.

7. Device according to claim 6, characterised in that the connection device (24) comprises a shell-like part (34) for receiving a stick-like electrode (36) which through a tongue-like, easily pliable part (32) is connected to a main part of the connecting device containing the screw hole (28).

8. Device according to the preamble of claim 1 or one of the claims 1 to 7, characterised in that the connection device (24) comprises a shell-like part (34) for receiving a stick-like electrode (36) which through a tongue-like, easily pliable part (32) is connected to a main part of the connection device containing a screw hole (28).

## Revendications

1. Dispositif implantable pour stimuler la croissance osseuse, comprenant une bobine réceptrice (10) qui présente au moins une première et une seconde borne; une enveloppe (14) de forme allongée, entourant la bobine réceptrice (10) et faite d'un matériau toléré par l'organisme; ainsi qu'au moins un dispositif de raccordement (24) servant à la connexion d'une électrode tissulaire (36) et qui est relié électriquement par un fil (22) isolé à la première borne de la bobine réceptrice (10), caractérisé en ce que la seconde borne de la bobine réceptrice (10) est couplée à une électrode tissulaire (44) allongée, ayant la forme d'une bande qui s'étend dans le sens de la longueur de l'enveloppe (14) et est disposée sur chant de manière à faire saillie à peu près perpendiculairement de cette enveloppe.

2. Dispositif selon la revendication 1, caractérisé en ce que l'électrode tissulaire (44) s'étend au-delà des extrémités longitudinales de l'enveloppe.

3. Dispositif selon la revendication 1 ou 2, caractérisé en ce que l'électrode tissulaire (44) est mécaniquement reliée solidement au système constitué de la bobine réceptrice (10) et de son enveloppe (14).

4. Dispositif selon une des revendications 1 à 3, caractérisé en ce que l'enveloppe possède approximativement la forme d'un rectangle plat et que le plan de l'électrode tissulaire (44) en forme de bande est perpendiculaire aux grands côtés du rectangle.

5. Dispositif selon une des revendications précédentes, caractérisé en ce qu'une électrode en forme de plaque est disposée sur le côté extérieur de la partie de l'enveloppe (14) à partir de laquelle fait saillie l'électrode tissulaire en forme de bande, à laquelle l'électrode en forme de plaque est reliée électriquement.

6. Dispositif selon la revendication 1 ou 2, caractérisé en ce que la seconde borne de la bobine réceptrice (10) est reliée à un contact (18) disposé libre sur un côté de l'enveloppe (14) et qu'un dispositif d'électrode (40) peut être glissé sur l'enveloppe (14), dispositif qui comporte une première partie (42) électriquement conductrice qui touche le contact (18) lorsque le dispositif d'électrode (40) est glissé en place, ainsi qu'une seconde partie (44) qui est reliée électriquement et mécaniquement à la première partie et constitue l'électrode tissulaire en forme de bande.

7. Dispositif selon la revendication 6, caractérisé en ce que le dispositif de raccordement (24) comporte une portion (34) en forme de douille, destinée à recevoir une électrode en barreau (36) et reliée par une portion (32) en forme de languette facilement flexible à la partie principale du dispositif de raccordement, laquelle contient un trou (28) pour le passage d'une vis.

8. Dispositif selon le préambule de la revendication 1 ou selon une des revendications 1 à 7, caractérisé en ce que le dispositif de raccordement (24) comporte une portion (34) en forme de douille, qui est destinée à recevoir une électrode en barreau (36) et qui est reliée par une portion (32) en forme de languette facilement flexible à une partie principale du dispositif de raccordement, laquelle contient un trou (28) pour le passage d'une vis.

FIG.1

FIG.3

FIG.2

FIG.4

FIG.5

FIG.6

FIG.7